# EUROPÄISCHE PATENTSCHRIFT

(11) **EP 1 370 831 B1**
(45) Veröffentlichungstag und Bekanntmachung des Hinweises auf die Patenterteilung: **26.08.2009**
(21) Anmeldenummer: 02724236.1
(22) Anmeldetag: 14.03.2002
(51) Int. Cl.: G01C 11/06, G01C 15/02, A61B 5/107, G01B 11/24

(54) **VERFAHREN UND ANORDNUNG ZUR PHOTOGRAMMETRISCHEN ERFASSUNG DER RAUMFORM EINES OBJEKTS**
METHOD AND ARRANGEMENT FOR PHOTOGRAPHICALLY DETECTING THE SPATIAL FORM OF AN OBJECT
PROCEDE ET DISPOSITIF POUR DETERMINER PAR PHOTOGRAMMETRIE LA FORME SPATIALE D'UN OBJET

(30) Priorität: 18.03.2001 DE 10113211
(43) Veröffentlichungstag der Anmeldung: 17.12.2003
(73) Patentinhaber: corpus.e AG, 70178 Stuttgart (DE)
(72) Erfinder: Massen, Robert, Prof. Dr., 78337 Öhningen-Wangen (DE)
(74) Vertreter: Prinz & Partner
(86) Internationale Anmeldenummer: PCT/EP2002/002875
(87) Internationale Veröffentlichungsnummer: WO 2002/074038

(56) Entgegenhaltungen:
- WO-A-97/14932
- DE-A- 19 626 889
- US-A- 4 825 263
- US-A- 5 911 126

## Beschreibung

Die Erfindung betrifft ein Verfahren und eine Anordnung zur photogrammetrischen Erfassung der Raumform eines Objekts.

Im Zusammenhang mit der sogenannten "Mass-Customization", d.h. der Produktion und dem Verkauf von individuell an die Körpermaße eines Kunden angepassten Produkten, ist die Erfassung der dreidimensionalen Raumform des Körpers oder von Körperteilen eine wichtige technisch zu lösende Aufgabe. Ist die dreidimensionale Raumform eines Körperteils, z.B. der Rumpf/Bein-Partie, eines Kunden bekannt, so können individuelle Kleidungsstücke wie eine Hose, ein Hemd usw. mit hoher Passform hergestellt werden.

Es sind eine Reihe von Körperscannern entwickelt worden, welche mit verschiedenen optischen Verfahren, z.B. Lasertriangulation, Stereo-Verfahren, Moiré-Verfahren etc. berührungslos die dreidimensionale Form des menschlichen Körpers oder von Körperteilen erfassen und digitalisieren, wobei die die Digitalisierung beschreibenden Datensätze dann für die automatisierte Produktion individuell angepasster Produkte bereitstellt werden. Diese Körperscanner arbeiten in der Regel mit technisch sehr aufwendigen und teuren Verfahren, die zudem eine optische Kalibrierung erfordern und daher nur von ausgebildetem Fachpersonal eingesetzt werden können.

Wesentliche kostengünstiger sind die sogenannten passiven Verfahren der Nahbereichsphotogrammetrie, da hier lediglich kalibrierte Photoapparate oder Digitalkameras, aber keine teuren Projektionseinheiten und genau kalibrierten mechanischen Aufbauten benötigt werden.

So ist z.B. aus dem Patent EP 0 760 622 "verfahren und Anordnung zur dreidimensionalen Erfassung der Raumform von Körpern und Körperteilen", dessen Anmelder und Erfinder Robert Massen ist, ein Verfahren bekannt, welches es mit einem sehr niedrigen technischen Aufwand gestattet, die 3D-Koordinaten eines Körpers oder eines Körperteils optisch zu erfassen. Hierzu wird der Körper mit einem speziell mit ortsgenauen Punktmarken markierten elastischen Überzug bekleidet und aus mehreren, sich überlappenden, aber sonst freihändig aufgenommenen Kamerapositionen photographiert. Durch eine photogrammetrische Auswertung der korrespondierenden Punktmarken in den einzelnen, sich überlappenden Bildbereichen der Aufnahmen kann ein 3D-Datensatz des Körperteils ermittelt werden.

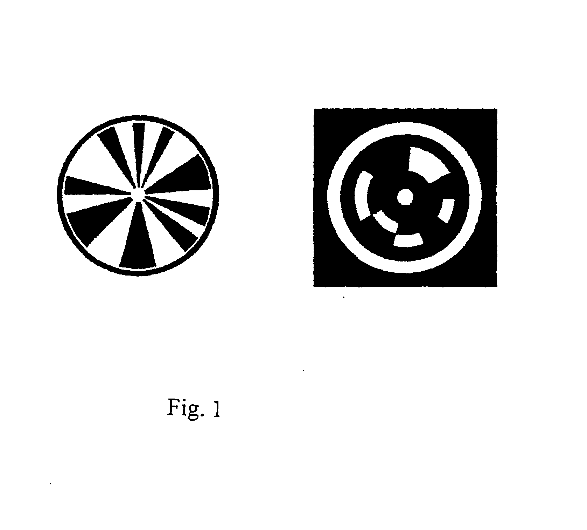

Um aus zwei sich überlappenden Bildaufnahmen mit den Methoden der Photogrammetrie eine 3D-Rekonstruktion des Körperteils aus den auf dem Körperteil oder einem über den Körperteil gezogenen Überzug angebrachten Punktmarken zu ermöglichen, müssen die Bildpunktkoordinaten der sog. homologen Punktmarken, d.h. der sich in den Aufnahmen jeweils entsprechenden Punktmarken, bestimmt werden. Dieser Vorgang wird auch mit Registrierung bezeichnet. Bei einer automatischen Registrierung müssen daher mit Verfahren der zweidimensionalen Bildverarbeitung aus zwei sich überlappenden Bildaufnahmen diejenigen Punktmarken gefunden werden, welche sich entsprechen. Bei bisherigen bekannten Verfahren wird dies durch die Verwendung von einzeln codierten Punktmarken erreicht. Es werden als Punktmarken z.B. Kreise, welche von radialen Segmenten, welche einen binären Code darstellen, umgeben sind (siehe hierzu den linken Teil der Fig. 1). Der Mittelpunkt der codierten Marke definiert dabei den Ort der photogrammetrischen Punktmarke. Jede Punktmarke ist durch die radialen Segmente eindeutig durch einen eigenen Code gekennzeichnet.

Bei den zum Stand der Technik gehörenden Verfahren wird mithilfe der automatischen Bildverarbeitung versucht, in zwei Bildern solche Punktmarken aufzufinden, ihren Code zu lesen und eine Liste der korrespondierenden Punktmarken, d.h. der homologen Punktmarken, zu erstellen. Liegt diese Liste fest, so kann mit den bekannten Verfahren der Bildorientierung und des Bündelausgleichs ein 3D-Datensatz erstellt werden, welcher die XYZ-Raumkoordinaten aller homologer Punktmarken enthält.

Bei der photogrammetrischen Vermessung von grossen Strukturen wie Schiffsrümpfen, Flugzeugteilen usw. spielt der Platzbedarf solcher codierter Punktmarken keine Rolle. Ein Nachteil der zum Stand der Technik gehörenden Verfahren zur photogrammetrischen Bestimmung der Raumform eines Objekts, die mit einzeln codierten und eine relativ große Fläche bedeckenden Punktmarken arbeiten, besteht jedoch darin, daß diese verfahren nicht zur Digitalisierung von sehr kleinen Objekten geeignet sind, die wegen der benötigten Raumpunktedichte mit vielen Punktmarken bedeckt werden müssen. Hier ist auf der Oberfläche der Objekte nicht genügend Platz, um die für eine gute photogrammetrische Vermessung benötigte Raumpunktdichte durch codierte Punktmarken, wie sie z.B. in der Fig. 1 dargestellt sind, zu erreichen.

In der deutschen Patentanmeldung mit dem Aktenzeichen 100 25 922.7 und dem Titel "Automatische photogrammetrische Digitalisierung von Körpern und Objekten", deren Anmelder und Erfinder Robert Massen ist, wird darüber hinaus vorgeschlagen, auf dem in der EP 0 760 622 beschriebenen Überzug Flächenmarken anzubringen, die jeweils mehrere Punktmarken umfassen und einen Hintergrund der Punktmarken bilden, wobei die Fläche der Flächenmarken eine bestimmte Farbe aufweist. Durch Verfahren der Farbbildverarbeitung können die in den verschiedenen photogrammetrischen Aufnahmen korrespondierenden Flächenmarken (Regionen) und daraus dann die korrespondieren Punktmarken (sogenannte homologe Bildpunkte) leicht automatisch ermittelt werden. Steht die Liste der homologen Bildpunkte bereit, so können mit den dem Fachmann der Photogrammetrie bekannten Methoden der Bildorientierung und des Bündelausgleichs die 3D-Daten des gesamten Körperteils berechnet werden.

Nachteilig ist bei diesem Verfahren, dass eine relativ große Anzahl verschiedener Farben zur Markierung benötigt wird. Die Farbtreue der eingesetzten Kameras muss daher ausreichend hoch und stabil sein, um diese Vielzahl unterschiedlicher Farben in einer automatischen Erkennung ausreichend unterscheiden zu können. Auch werden an die Konstanz und Farbtreue der Beleuchtung höhere Anforderungen gestellt als bei einer Markierungstechnik, welche mit nur sehr wenigen, sich im Farbraum deutlich unterscheidenden Farben auskommen würde. Ein weiterer Nachteil dieses Verfahrens ist die relativ hohe geometrische Auflösung, welche benötigt wird, um die als Marken verwendeten Farbkanten und Farbecken optisch aufzulösen.

Beide Anforderungen, die hohe Farbtreue und die hohe geometrische Auflösung, lassen sind nicht mit einfachen und sehr preiswerten Kameras erfüllen, wie z.B. mit den auf CMOS-Bildsensoren basierenden Webcams oder den Bildsensoren, welche die in den zukünftigen Mobiltelephonen und sogenannten Personal Organizern (Taschencomputern) eingebaut sind. Damit können solche sehr einfachen und preiswerten Bildaufnahmegeräte nicht für die beschriebene einfache Digitalisierung von Körperteilen eingesetzt werden.

Es besteht daher ein technisches und wirtschaftliches Interesse daran, ein Verfahren und eine Anordnung zur photogrammetrischen Erfassung der Raumform eines Objekts zu schaffen, welches eine verbesserte Markierungsmethode aufweist, die eine preiswerte und einfache Vermessung auch von kleinen Objekten erlaubt.

Darüber hinaus besteht ein Interesse an einem Verfahren und einer Anordnung zur photogrammetrischen Erfassung der Raumform eines Objekts, welche bei der Markierung mit nur wenigen unterschiedlichen Farben und nur relativ groben Strukturen auskommen, damit die automatische Bestimmung homologer Bildpunkte aus mehreren, sich überlappenden Bildaufnahmen unter Verwendung von preiswerten, wenig farbtreuen und niedrig auflösenden Kameras und Bildgebern sowie bei einer Beleuchtung möglich wird, die in bezug auf die Farbe wenig definiert sein kann. Dadurch würde sich das Verfahren auch unter Verwendung von z.B. heute üblichen preiswerten und in Bezug auf die Farbtreue und Auflösung eher schwach spezifizierten Webcams (Web-Kameras) oder Digitalkameras einsetzen lassen.

Gemäß der Erfindung wird das auf diesem technischen Gebiet oben dargestellte Interesse durch ein Verfahren zur photogrammetrischen Erfassung der Raumform eines Objekts befriedigt, bei dem
auf der Oberfläche des Objekts mehrere photogrammetrische Punktmarken und mehrere Verbindungsmarken, die jeweils eine Teilmenge der mehreren Punktmarken miteinander verbinden, angebracht werden, wobei es mindestens zwei verschiedene Arten von Punktmarken gibt, die sich durch ihre optische Gestaltung voneinander unterscheiden, und
einige der entlang einer Verbindungsmarke angebrachten Punktmarken so ausgebildet sind, daß die Abfolge ihrer optischen Gestaltungen einen vorherbestimmten die jeweilige Verbindungsmarke charakterisierenden Code ergibt, mehrere photogrammetrische Aufnahmen des Objekts aus verschiedenen Ansichten gemacht werden,
eine Bildverarbeitung der Aufnahmen durchgeführt wird, bei der zunächst die sich in den Aufnahmen jeweils entsprechenden Verbindungsmarken anhand ihres jeweiligen Codes einander zugeordnet werden und dann mithilfe der Verbindungsmarkenzuordnung in den Aufnahmen die durch die jeweilige Verbindungsmarke miteinander verbundenen Punktmarken einander zugeordnet werden, und
anhand der Punktmarkenzuordnung die Raumform des Objekts mit einem photogrammetrischen Auswerteverfahren ermittelt wird.

Ferner wird das oben beschriebene Interesse durch eine Anordnung zur photogrammetrischen Erfassung der Raumform eines Objekts befriedigt, die ein Aufnahmesystem zur Erstellung photogrammetrischer Aufnahmen verschiedener Ansichten des Objekts und ein System zur Verarbeitung und Auswertung der Aufnahmen sowie zur Ermittlung der Raumform des Objekts umfaßt und dadurch gekennzeichnet ist, daß die Anordnung darüber hinaus auf der Oberfläche des Objekts mehrere photogrammetrische Punktmarken und mehrere Verbindungsmarken aufweist, die jeweils eine Teilmenge der mehreren Punktmarken miteinander verbinden, wobei es mindestens zwei verschiedene Arten von Punktmarken gibt, die sich durch ihre optische Gestaltung voneinander unterscheiden, und einige der entlang einer Verbindungsmarke angebrachten Punktmarken so ausgebildet sind, daß die Abfolge ihrer optischen Gestaltungen einen vorherbestimmten die jeweilige Verbindungsmarke charakterisierenden Code ergibt.

Dadurch, daß gemäss der Erfindung nicht die Punktmarken selbst einzeln codiert sind, sondern eine charakterisitsche Abfolge von Punktmarken verschiedener Art einen Code ergibt, lassen sich auch sehr kleine Objekte, die eine hohe Punktmarkendichte erfordern, leicht automatisch photogrammetrisch erfassen.

Vorteilhafte Weiterbildungen der Erfindung sind in den abhängigen Patentansprüchen gekennzeichnet.

Weitere Merkmale und Vorteile der Erfindung ergeben sich aus der folgenden Beschreibung eines Ausführungsbeispiels anhand der Zeichnungen. In den Zeichnungen zeigen:
Fig. 1 zwei verschiedene zum Stand der Technik gehörende codierte Punktmarken;
Fig. 2 eine Seitenansicht eines mit dem erfindungsgemäßen Verfahren zu digitalisierenden Knies mit einem Überzug und einer Kamera in zwei verschiedenen Aufnahmepositionen;
Fig. 3 das in der Fig. 2 dargestellte Knie in einer Draufsicht, wobei die radial um das Knie verteilten Aufnahmepositionen einer Kamera dargestellt sind;
Fig. 4 einen gemäß einer ersten Ausführungsform der Erfindung photogrammetrisch markierten Knie-Überzug;
Fig. 5a den in der Fig. 4 dargestellten Knie-Überzug in einer ersten Aufnahmeposition;
Fig. 5b den in der Fig. 4 dargestellten Knie-Überzug in einer zweiten Aufnahmeposition;
Fig. 6 die bei einer weiteren Ausführungsform der Erfindung verwendete photogrammetrische Markierungsform;
Fig. 7 die bei einer weiteren Ausführungsform der Erfindung verwendete photogrammetrische Markierungsform.

Die Erfindung wird beispielhaft, was nicht einschränkend zu verstehen ist, anhand der photogrammetrischen Erfassung der Raumform einer Kniepartie zwecks automatischer Herstellung einer Knieorthese beschrieben.

Beispielhaft sei angenommen, dass das Knie mit einem elastischen Überzugs bekleidet ist, auf dem photogrammetrische Marken angebracht sind. Die photogrammetrischen Marken können jedoch auch durch eine Vielzahl anderer Verfahren angebracht werden. Das Anbringen der Marken auf dem Objekt kann z.B. auch durch Auftragen mit Farbe, z.B. Schminke, direkt auf das Objekt, durch Projizieren mithilfe eines Projektionssystems oder durch Aufkleben von selbstklebenden Folien, auf denen die Marken angebracht sind, erfolgen.

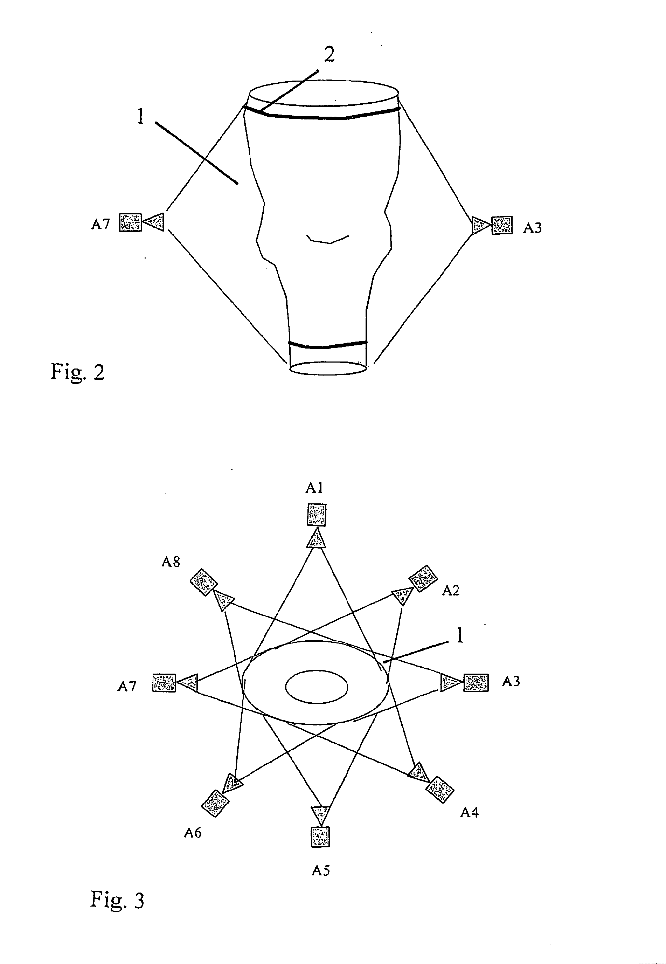

Das beispielhaft zu digitalisierende und in der Fig. 2 dargestellte Knie 1 mit den anschliessenden Oberschenkel- und Unterschenkelpartien hat ungefähr eine zylindrische Grundform. Die erforderlichen, sich überlappenden photographischen Aufnahmen werden in diesem Fall zweckmäßigerweise in Form von Rundansichten des zylindrischen Körperteils erstellt, wobei zur Sicherstellung einer ausreichenden Überlappung etwa 8 bis 10 über den Umfang verteilte Aufnahmen benötigt werden.

Fig. 3 zeigt, wie die mit einem markierten elastischen Überzug (2 in der Fig. 2) bekleidete Kniepartie 1 eines Patienten, aus acht radialen Ansichten A1 bis A8 mit einer Kamera aufgenommen wird. Die Fig. 2 zeigt eine Seitenansicht des Knies, bei der die Kamera nur in den Positionen A3 und A7 dargestellt ist. Hierbei ist es für die photogrammetrische Auswertung lediglich wichtig, dass der angulare Überlappungsbereich der einzelnen Aufnahmen eine ausreichende Zahl von homologen , d.h. sich entsprechenden photogrammetrischen Punktmarken aufzeigt. Die Kamera kann beispielsweise für eine solche Rundumaufnahmesequenz in der gleichen Höhe per Hand um das Bein herum geführt werden.

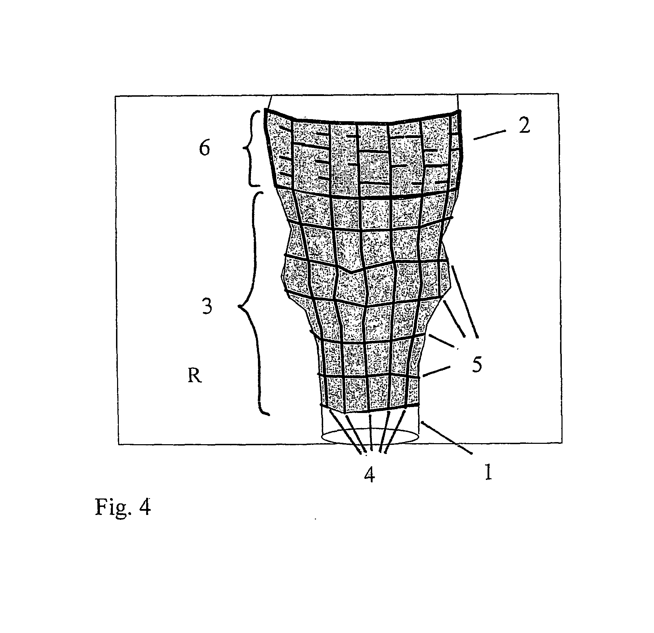

Fig. 4 zeigt den Kniebereich 1 mit dem elastischen Überzug 2, auf dem verschiedene photogrammetrische erfindungsgemäße Markierungen angebracht sind.

In seinem unteren Bereich 3 ist der Überzug 2 in einer ersten Farbe eingefärbt, die einen deutlichen Kontrast zu dem von den Kameras ebenfalls erfaßten Raumhintergrund R bildet und in der Fig. 4 durch einen hellen Grauton dargestellt ist. Als Farbe wird zweckmäßigerweise eine im gewöhnlichen Umfeld eher seltene Farbe wie z.B. Rosa gewählt. Auf dem hellen Grauton sind Gitterlinien angebracht, wobei die Schnittpunkte der vertikalen und horizontalen Gitterlinien 4 bzw. 5 ortsgenaue photogrammetrische Punktmarken bilden, die im folgenden auch als nicht zur Codierung beitragende Punktmarken bezeichnet werden, da sie sich nicht voneinander unterscheiden lassen und keinen Code beinhalten und auch nicht zur Codierung dienen können.
In seinem oberen Bereich 6 ist der Überzug 2 in einer zweiten Farbe eingefärbt, die einen deutlichen Kontrast zum Raumhintergrund R und einen deutlichen Kontrast zur ersten Farbe des unteren Bereichs 3 bildet (z.B. Hellgrün). Die zweite Farbe ist in der Fig. 4 durch einen dunklen Grauton dargestellt. Die vertikalen Linien 4 der Gitterstruktur des unteren Bereichs 5 verlaufen auch in den oberen Bereich hinein.
In dem oberen Bereich 6 werden die Punktmarken dadurch gebildet, daß eine relativ kurze horizontale Linie auf eine vertikale Linie 4 zuläuft und an der vertikalen Linie endet, wobei der Auftreffpunkt die ortsgenaue Punktmarke definiert. Wie in der Fig. 4 zu erkennen ist, gibt es dabei zwei verschiedene Arten von Punktmarken, nämlich solche, bei denen die relativ kurze horizontale Linie von links auf die vertikale Linie der Gitterstruktur zuläuft und solche, bei denen die relativ kurze horizontale Linie von rechts auf die vertikale Linie der Gitterstruktur zuläuft.
Entlang jeder vertikalen Linie 4 der Gitterstruktur, die im folgenden auch als Verbindungsmarke bezeichnet wird, da sie verschiedene Punktmarken miteinander verbindet, sind dabei in dem oberen Farbbereich 6 vier Punktmarken angeordnet, deren Abfolge, d.h. die Abfolge der Anordnung ihrer horizontalen Linien (z.B. von links, von rechts, von links, von links auf die vertikale Linie zulaufend), die jeweilige Verbindungsmarke eindeutig kennzeichnet. Die Verbindungsmarken verbinden in dem oberen Bereich 6 liegende Punktmarken mit den im unteren Bereich 5 liegenden Punktmarken.
Mithilfe einer einfachen bildpunktweise durchgeführten Farbklassifikation können aus jeder Bildaufnahme bereits automatisch die Region "KNIE" anhand der ersten Farbe vom Hintergrund segmentiert sowie die Coderegion 6 innerhalb der Region "KNIE" anhand der zweiten Farbe lokalisiert werden. Es ist dem Fachmann der Bildverarverarbeitung bekannt, wie solche automatischen Farbsegmentierungen robust durchgeführt werden können (siehe z.B. Robert Massen: "Form und Farbe: Farbbildverarbeitung für die industrielle Überwachung in Echtzeit" in : Maschinelles Sehen, Europa-Fachpresse Verlag 1990, ISBN 3-87207-004-5).

Die zur photogrammetrischen Auswertung benötigten Marken (Punkt- und Verbindungsmarken) werden durch kontraststarke schwarze Linien dargestellt, wobei die Breite dieser Linien so gross gewählt ist, dass sie auch noch von preiswerten, in der Regel nur schwach auflösenden Kameras abgebildet werden können. Die Kreuzungspunkte der Linien bzw. im Code-Bereich 6 die Auftreffpunkte stellen dabei die eigentlichen photogrammetrischen Punktmarken dar. Die Lage der Punktmarken kann mit Hilfe von Interpolationsverfahren mit hoher Genauigkeit bestimmt werden (Sub-Pixel-Interpolation), so dass trotz einer schlechten geometrischen Kameraauflösung ortsgenaue Punktmarkenkoordinaten gemessen werden können. So lässt sich mit solchen, dem Fachmann der Bildverarbeitung bekannten Sub-Pixel- Interpolationen die XY-Lage des Schwerpunkts einer Gitterkreuzung mit einer Genauigkeit bestimmen, welche etwa 5 bis 10 mal höher ist als die Grösse des abbildenden Bildpunktes.

Die Verbindungslinien zwischen den Kreuzungspunkten dienen als "optische Gleise", entlang denen die automatische Bildauswertung gezielt von Kreuzungspunkt zu Kreuzungspunkt fortschreiten kann. Sie stellen damit sowohl in vertikaler als auch in horizontaler Richtung die Nachbarschaftsbeziehungen zwischen den zur Codierung beitragenden oder nichtcodierten Marken her.

Um aus zwei sich überlappenden Bildaufnahmen mit den Methoden der Photogrammetrie eine 3D-Rekonstruktion der auf dem Knie-Überzug angebrachten befestigten Marken zu ermöglichen, müssen die Bildpunktkoordinaten der homologen Punktmarken bestimmt werden, was auch als Registrierung bezeichnet wird. Bei einer automatischen Registrierung müssen mit Verfahren der 2-dimensionalen Bildverarbeitung aus zwei sich überlappenden Bildaufnahmen diejenigen Marken gefunden werden, welche sich entsprechen. Beim Stand der Technik wird dies durch Verwendung von einzeln codierten und relativ großflächigen Punktmarken erreicht, wie sie z.B. in der Fig. 1 dargestellt sind. Es werden dann mithilfe der automatischen Bildverarbeitung gemäß dem Stand der Technik in zwei Bildern Punktmarken aufgefunden, es wird ihr Code gelesen und es wird dann eine Liste der korrespondierenden Marken, d.h. der homologen Punktmarken, erstellt. Liegt diese Liste fest, so kann mit den bekannten Verfahren der Bildorientierung und des Bündelausgleichs ein 3D-Datensatz erstellt werden, welcher die XYZ-Raumkoordinaten aller homologer Punktmarken enthält.

Erfindungsgemäss wird dagegen eine charakteristische Abfolge von einfachen und sehr kleinen verschiedenartigen Punktmarken dazu verwendet, Verbindungsmarken (z.B. Linien, die die Punktmarken miteinander verbinden) zu charakterisieren, auf denen jeweils eine bestimmte Teilmenge der Punktmarken und insbesondere die Punktmarken, die nicht zur Codierung beitragen, hintereinander angeordnet ist. Die eindeutige Zuordnung der nicht zur Codierung beitragenden Punktmarken kann dabei aus den Nachbarschaftsbeziehungen zwischen den codierten Marken hergestellt werden.

Erfindungsgemäß wird weiterhin eine platzsparende Codierung verwendet, welche so aufgebaut ist, dass die einzelnen Codestellen der codierten Marke jeweils als eine photogrammetrische Punktmarke verwendet werden können. Hierbei wird z.B. ein vierstelliger Code nicht nur durch eine Punktmarke, sondern aus vier Punktmarken gebildet. Beim üblichen Verfahren der mit radialen Segmenten codierten Punktmarken (siehe links in der Fig. 1) gibt es pro Code (d.h. für alle Codeelemente zusammen genommen) nur eine einzige Punktmarke. Das erfindungsgemässe Verfahren multipliziert daher die Anzahl der verfügbaren Punktmarken mit dem Faktor N, wobei N die Anzahl der Codestellen (BIT-Stellen bei einem binären Code) bezeichnet. Damit kann ein Körperteil wesentlich dichter digitalisiert werden, da er dichter mit codierten Punktmarken überzogen werden kann.

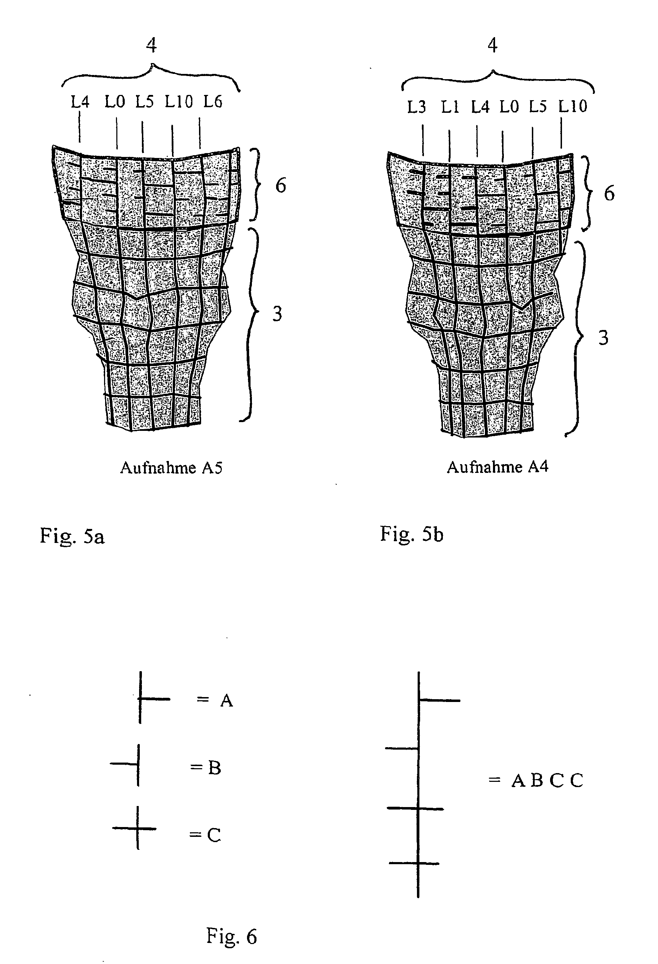

Fig. 5a und 5b zeigen zwei aus den verschiedenen Aufnahmepositionen A5 und A4 (siehe hierzu die Fig. 3) erstellte Aufnahmen des mit dem Überzug versehenen Knies. Bei dem zu digitalisierenden Knie, bei dem es sich um einen ungefähr zylinderförmigen Körper handelt, und bei dem die automatische Zuordnung korrespondierender Bildpunkte aus sich überlappenden Umfangsaufnahmen ermittelt werden soll, werden die vertikalen Gitterlinien (die entlang der Längsachse des Beines orientierten Linien ) Li in der mit der ersten Farbe gekennzeichneten Coderegion 6 durch einen binären Code mit den beiden Codeelementen:
- horizontale Abzweigung von der Gitterlinie nach links = logisch NULL = O
- horizontale Abzweigung von der Gitterlinie nach rechts = logisch EINS = L gekennzeichnet.

Die Aufnahme A5 in der Fig. 5a zeigt einen vierstelligen Binärcode welcher jede vertikale Linie (Verbindungsmarke 4) eindeutig codiert:
Linie L 4 = ( O L O O
Linie L 0 = ( O O O O )
Linie L 5 = ( O L O L )
Linie L 10 = ( L O L O )
Linie L 6 = ( O L L O )

Das erste Codebit wird z.B. durch die erste Gitterverzweigung welche von oben ausgehend angetroffen wird, dargestellt. Durch den markanten Farbübergang zwischen der mit der ersten Farbe flächig eingefärbten Coderegion 6 und der mit der zweiten Farbe flächig eingefärbten übrigen unteren Knieregion 3 kann automatisch der Aufsetzpunkt für die Decodierung dieses Codes mit Verfahren der Farb- und Grauwertbildverarbeitung gefunden werden, auch wenn die Kamera zwischen den Aufnahmen verdreht oder gekippt wird oder sonst in einer unbekannte Aufnahmeposition gerät.

In der Fig. 5b ist die Bildaufnahme A4 des Knies mit Überzug dargestellt, die aus einer radial um 45 Grad gegenüber der Aufnahme A5 versetzten Aufnahmerichtung entstanden ist.

Durch automatisches Ablesen des Codes der Verbindungsmarken (d.h. der vertikalen Linien 4) können sofort die sich in den beiden Aufnahmen A5 und A4 entsprechenden Verbindungsmarken, d.h. die homologen vertikalen Linien 4, bestimmt werden.
Bei der photogrammetrischen Auswertung wird nun zunächst eine Bildverarbeitung der Aufnahmen durchgeführt wird, bei der zunächst die sich in den Aufnahmen jeweils entsprechenden Verbindungsmarken 4 anhand ihres jeweiligen Codes einander zugeordnet werden und dann mithilfe der Verbindungsmarkenzuordnung in den Aufnahmen die durch die jeweilige Verbindungsmarke miteinander verbundenen Punktmarken einander zugeordnet werden, wobei anhand der Punktmarkenzuordnung die Raumform des Objekts mit einem photogrammetrischen Auswerteverfahren ermittelt wird.
Besonders vorteilhaft ist es bei diesem Ausführungsbeispiel der Erfindung, daß die Codestelle "Verzweigung" gleichzeitig als photogrammetrische Punktmarke dienen kann. Der Schwerpunkt einer Links- oder Rechtsverzweigung lässt sich bei der Bildauswertung ebenso genau ermitteln wie der Schwerpunkt eines durchgehenden Gitterkreuzes. Damit ist diese Codierung im Gegensatz zu den bekannten Verfahren extrem platzsparend.

Ein besonderer Vorteil der Erfindung besteht darin, dass jede Codestelle eine Punktmarke darstellt. Damit verbraucht ein solcher Code wenig von dem für die Digitalisierung benötigten Platz, weil er auf mehrere kompakte Punktmarken verteilt ist.

Selbstverständlich muss dieser Code nicht auf einen binären Code beschränkt sein. Fig. 6 zeigt beispielsweise die Codierung der Verbindungsmarken (vertikalen Linien) mit einem ternären Code, welcher aus den Elementen:
- horizontale Abzweigung nach links = A
- horizontale Abzweigung nach rechts = B
- Abzweigung nach links und nach rechts = C
besteht.
Mit lediglich 3 verschiedenen Kreuzungsarten ( = Codestellen ) können bereits 3*3 = 9 Verbindungsmarken (Linien) eindeutig codiert werden, so dass ein solcher Code nochmals wesentlich weniger Platz beansprucht als der binäre Code.

Es kann unter Umständen vorteilhaft sein, über mehr Codes zu verfügen als für die eindeutige Kennzeichnung sämtlicher Verbindungsmarken benötigt werden, da zusätzliche Codes zur Codesicherung, Fehlererkennung und Fehlerkorrektur verwendet werden können. Die Verwendung und Auslegung hierbei auftretender redundanter Codes für eine automatische Fehlerkorrektur und Fehlererkennung von beim Codelesen auftretenden Fehlern ist dem Fachmann der Codierung bekannt.

Es ist ersichtlich, daß das erfindungsgemäße Prinzip bei einer Vielzahl von verschiedenen Punktmarken verwendung finden kann, wobei die Punktmarken nicht notwendigerweise aus Kreuzen oder aufeinandertreffenden Linien zu bestehen brauchen. Es müssen lediglich mindestens zwei verschiedene Arten von Punktmarken bereitgestellt werden, wobei die genaue Form der Punktmarken keine Rolle spielt, sie müssen nur ihrer Funktion als Punktmarken folgend, geeignet sein, den Ort eines Punktes auf dem Objekt festzulegen.

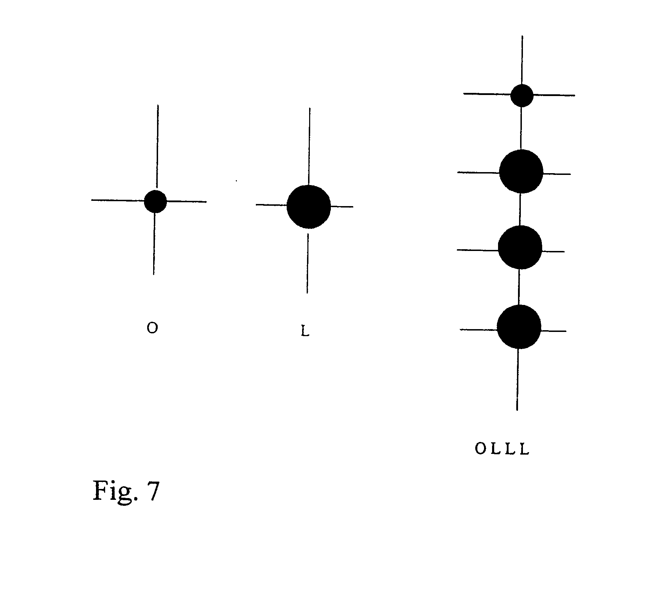

So ist in der Fig. 7 eine Ausführungsform der Erfindung dargestellt, bei der schwarze Punkte mit unterschiedlichem Durchmesser als Punktmarken eingesetzt werden, wobei ein Punkt mit einem kleinen Durchmesser (links in der Fig. 7) eine logische Null und ein Punkt mit einem großen Durchmesser (in der Mitte der Fig. 7) eine logische Eins des Codes markiert.
So ergibt sich z.B. rechts in der Fig. 7 der Code "0 L L L L", durch Hintereinanderlegen von einem kleinen und drei großen schwarzen Kreisen.
Selbstverständlich müssen als Verbindungsmarken nicht unbedingt vertikale gerade Linien verwendet werden, die die Punktmarken miteinander verbinden. Es können z.B. ebenso horizontale Linien oder gekrümmte Linien verwendet werden. Wichtig ist nur, daß durch die Verbindungsmarken eine Verbindung zwischen den Punktmarken hergestellt wird, die der Verbindungsmarke zuzuordnen sind. Es ist daher auch denkbar, daß die Verbindungsmarken selbst aus Punkten bestehen.

Damit sind erfindungsgemäß drei Aufgaben mit dieser erfindungsgemässen Markierung gelöst: es wird eine grossflächige, auch von einer schlecht auflösenden Kamera erfassbare Codierung einer Struktur (im beschriebenen Beispiel dicke Gitterlnien) möglich, es kann diese Codierung gleichzeitig als eine Vielzahl ortsgenauer photogrammetrischer Punktmarken ausgenutzt werden und es kann eine automatische Bestimmung homologer Punktmarken mithilfe der Bestimmung homologer Verbindungsmarken erfolgen.

Bei anderen Ausführungsformen der Erfindung können natürlich die zur Unterscheidung zwischen zur Codierung beitragenden Punktmarken und nicht zur Codierung beitragenden Punktmarken angebrachten Flächen durch verschiedenste optische Gestaltungen voneinander abgehoben werden. Diese optische Gestaltung muß nicht unbedingt in einer unterschiedlichen Farbe bestehen, sondern kann auch in einem charakteristischen Grauwert, einer charakteristischen Textur, d.h. einem charakteristischen Muster oder einer charakterisitschen Struktur, einem charakteristischen Glanz, einer auf der Fläche aufgebrachten Oberflächenbeschichtung mit charakteristischem Polarisationsgrad, charakteristischen Fluoreszenzeigenschaften, bestimmten spektralen Signalturen, einem bestimmten lokalen Glanz, einer bestimmten lokalen Temperatur oder einer charakteristischen Kombination der aufgezählten optischen Gestaltungsmerkmale bestehen.

Auch können die zur photogrammetrischen Markierung aufgebrachten kontraststarken Strukturen sich durch Reflexionseigenschaften in bezug auf einen außerhalb des sichtbaren Bereichs liegenden Spektralbereich auszeichnen. Sie können z.B. aus nur im nahen Infrarot-Bereich, im Bereich des Ultravioletten oder in einem sonstigen, für das menschliche Auge nicht sichtbaren Bereich der Wellenlängen gegenüber dem Hintergrund kontrastieren. So lassen sich z.B. elastische Überzüge herstellen, bei denen die Markierung unsichtbar ist, bzw. von einer sichtbaren, für das menschliche Auge bestimmten Farbgebung überdeckt wird. Diese können z.B. Schwimmanzüge sein, die zum einen mit einem für das menschliche Auge sichtbaren "ästhetischen" Muster gestaltet sein können, und zusätzlich mit einer unsichtbaren photogrammetrisch erfaßbaren Oberflächenmarkierung aus Punkt- und Verbindungsmarken.

## Patentansprüche

1. Verfahren zur photogrammetrischen Erfassung der Raumform eines Objekts (1), bei dem
auf der Oberfläche des Objekts (1) mehrere photogrammetrische Punktmarken und mehrere Verbindungsmarken (4), die jeweils eine Teilmenge der mehreren Punktmarken miteinander verbinden, angebracht werden, wobei es mindestens zwei verschiedene Arten von Punktmarken gibt, die sich durch ihre optische Gestaltung voneinander unterscheiden, und
einige der entlang einer Verbindungsmarke (4) angebrachten Punktmarken so ausgebildet sind, daß die Abfolge ihrer optischen Gestaltungen einen vorherbestimmten die jeweilige Verbindungsmarke charakterisierenden Code ergibt, mehrere photogrammetrische Aufnahmen des Objekts aus verschiedenen Ansichten (A1, A2, ..., A8) gemacht werden,
eine Bildverarbeitung der Aufnahmen durchgeführt wird, bei der zunächst die sich in den Aufnahmen jeweils entsprechenden Verbindungsmarken (4) anhand ihres jeweiligen Codes einander zugeordnet werden und dann mithilfe der Verbindungsmarkenzuordnung in den Aufnahmen die durch die jeweilige Verbindungsmarke miteinander verbundenen Punktmarken einander zugeordnet werden, und
anhand der Punktmarkenzuordnung die Raumform des Objekts (1) mit einem photogrammetrischen Auswerteverfahren ermittelt wird.

2. Verfahren nach Anspruch 1, bei dem die Verbindungsmarken (4) aus Linien bestehen.

3. Verfahren nach Anspruch 2, bei dem die Linien gerade sind.

4. Verfahren nach Anspruch 2, bei dem die Linien gekrümmt sind.

5. Verfahren nach einem der vorhergehenden Ansprüche 2 bis 4, bei dem die Punktmarken jeweils durch zwei aufeinandertreffende Linien definiert werden, von denen eine zu einer Verbindungsmarke (4) gehört, die eine Teilmenge der Punktmarken miteinander verbindet.

6. Verfahren nach Anspruch 5, bei dem die unterschiedliche optische Gestaltung der Punktmarken darin besteht, daß manche Punktmarken von der einen Seite der eine Verbindungsmarke bildenden Linie auf diese zulaufen und an dieser enden und manche anderen Punktmarken von der anderen Seite der eine Verbindungsmarke bildenden Linie auf diese zulaufen und an dieser enden, wobei die Abfolge von von einen und der anderen Seite zulaufenden Punktmarken den die Verbindungsmarke charakterisierenden Code ergibt.

7. Verfahren nach Anspruch 5, bei dem die unterschiedliche optische Gestaltung der Punktmarken darin besteht, daß manche Punktmarken einer Seite auf eine eine Verbindungsmarke bildende Linie zulaufen und an der Linie enden und manche Punktmarken die Verbindungsmarke bildende Linie kreuzen, wobei die Abfolge von von einer Seite zulaufenden Punktmarken und kreuzenden Punktmarken den die Verbindungsmarke charakterisierenden Code ergibt.

8. Verfahren nach einem der Ansprüche 1 bis 4, bei dem die Punktmarken durch Kreise gebildet werden.

9. Verfahren nach Anspruch 8, bei dem die unterschiedliche optische Gestaltung der Punktmarken darin besteht, daß die Punkte unterschiedliche Durchmesser haben.

10. Verfahren nach einem der vorhergehenden Ansprüche, bei dem die Punktmarken, die einen Code bilden mit einer in einer ersten Art optisch gestalteten Fläche hinterlegt sind und die übrigen Punktmarken, die keinen Code bilden, mit einer in einer zweiten sich von der ersten Art unterscheidenden Art gestalteten Fläche hinterlegt sind.

11. Verfahren nach Anspruch 10, bei dem die unterschiedliche optische Gestaltung der Flächen darin besteht, daß die Flächen unterschiedliche Farben haben.

12. Verfahren nach einem der vorhergehenden Ansprüche, bei dem das Anbringen der Punkt- und Verbindungsmarken (4) auf dem Objekt (1) in der Weise erfolgt, daß ein elastischer Überzug (2), auf dem die Marken aufgebracht sind, über das Objekt gezogen wird.

13. Verfahren nach einem der Ansprüche 1 bis 11, bei dem das Anbringen der Punkt- und Verbindungsmarken (4) auf dem Objekt (1) in der Weise erfolgt, daß die Marken auf das Objekt (1) mit Farbe aufgebracht werden.

14. Verfahren nach einem der Ansprüche 1 bis 11, bei dem das Anbringen der Punkt- und Verbindungsmarken (4) auf dem Objekt (1) in der Weise erfolgt, daß die Marken auf das Objekt (1) projiziert werden.

15. Verfahren nach einem der Ansprüche 1 bis 11, bei dem das Anbringen der Punkt- und Verbindungsmarken (4) auf dem Objekt (1) in der Weise erfolgt, daß auf die Oberfläche des Objekts (1) selbstklebende Folien geklebt werden, auf denen die Marken aufgebracht sind.

16. Anordnung zur photogrammetrischen Erfassung der Raumform eines Objekts mit einem Aufnahmesystem zur Erstellung photogrammetrischer Aufnahmen verschiedener Ansichten des Objekts (1) und einem System zur Verarbeitung und Auswertung der Aufnahmen sowie zur Ermittlung der Raumform des Objekts, **dadurch gekennzeichnet, daß** die Anordnung darüber hinaus auf der Oberfläche des Objekts mehrere photogrammetrische Punktmarken und mehrere Verbindungsmarken (4) aufweist, die jeweils eine Teilmenge der mehreren Punktmarken miteinander verbinden, wobei es mindestens zwei verschiedene Arten von Punktmarken gibt, die sich durch ihre optische Gestaltung voneinander unterscheiden, und einige der entlang einer Verbindungsmarke (4) angebrachten Punktmarken so ausgebildet sind, daß die Abfolge ihrer optischen Gestaltungen einen vorherbestimmten die jeweilige Verbindungsmarke charakterisierenden Code ergibt.

17. Anordnung nach Anspruch 16, die darüber hinaus einen elastischen Überzug (2) aufweist, auf dem die Punkt- und Verbindungsmarken (4) angebracht sind und der so ausgebildet ist, daß er über das Objekt (1) gezogen werden kann und sich dabei an die Form des Objekts (1) anpaßt.

18. Anordnung nach Anspruch 16, die darüber hinaus eine oder mehrere selbstklebende Folien aufweist, auf denen die Punkt-und Verbindungsmarken (4) angebracht sind und die so ausgebildet sind, daß sie, wenn sie auf die Oberfläche des Objekts (1) geklebt sind, eng an dem Objekt (1) anliegen.

19. Anordnung nach Anspruch 16, die darüber hinaus ein Projektionssystem umfaßt, das so ausgebildet ist, daß damit die Punkt- und Verbindungsmarken (4) auf das Objekt (1) projiziert werden können.

20. Verfahren nach einem der Ansprüche 1 bis 15 oder Anordnung nach einem der Ansprüche 16 bis 19, bei dem bzw. bei der das Objekt der Körperteil eines Menschen ist.

## Claims

1. A method of detecting the 3D shape of an object (1) by photogrammetry, in which
a plurality of photogrammetric point markers and a plurality of connecting markers (4) are provided on the surface of the object (1), each connecting marker connecting a subset of the plurality of point markers with each other, with at least two different types of point markers existing that differ from each other in their optical configuration, and
some of the point markers provided along a connecting marker (4) are formed in such a way that the sequence of their optical configurations results in a predetermined code that characterizes the respective connecting marker,
a plurality of photogrammetric images of the object are taken from different views (A1, A2, ..., A8),
an image processing of the images is performed, in which first the connecting markers (4) mutually corresponding to each other in the images are associated with one another using their respective code, and then the point markers connected with each other by the respective connecting marker are associated with one another with the aid of the connecting marker association in the images, and
using the point marker association, the 3D shape of the object (1) is determined by means of a photogrammetric evaluation process.

2. The method as claimed in claim 1, in which the connecting markers (4) consist of lines.

3. The method as claimed in claim 2, in which the lines are straight.

4. The method as claimed in claim 2, in which the lines are curved.

5. The method as claimed in any of the preceding claims 2 to 4, in which the point markers are each defined by two lines meeting each other, one of which is part of a connecting marker (4) that connects a subset of the point markers with one another.

6. The method as claimed in claim 5, in which the different optical configuration of the point markers consists in that some point markers run from one side of the line forming a connecting marker toward such line to end thereat, and some other point markers run from the other side of the line forming a connecting marker toward such line to end thereat, the sequence of point markers running from the one side or from the other side resulting in the code characterizing the connecting marker.

7. The method as claimed in claim 5, in which the different optical configuration of the point markers consists in that some point markers run from one side toward a line forming a connecting marker to end at the line, and some point markers cross the line forming a connecting marker, the sequence of point markers running from one side and crossing point markers resulting in the code characterizing the connecting marker.

8. The method as claimed in any of claims 1 to 4, in which the point markers are formed by circles.

9. The method as claimed in claim 8, in which the different optical configuration of the point markers consists in that the points have different diameters.

10. The method as claimed in any of the preceding claims, in which the point markers that form a code are backed by an area having a first type of optical configuration, and the remaining point markers that do not form a code are backed by an area having a second type of configuration that differs from the first type.

11. The method as claimed in claim 10, in which the different optical configuration of the areas consists in that the areas have different colors.

12. The method as claimed in any of the preceding claims, in which the point and connecting markers (4) are provided on the object (1) by pulling an elastic envelope (2) having the markers applied thereto over the object.

13. The method as claimed in any of claims 1 to 11, in which the point and connecting markers (4) are provided on the object (1) by applying the markers onto the object (1) using paint.

14. The method as claimed in any of claims 1 to 11, in which the point and connecting markers (4) are provided on the object (1) by projecting the markers onto the object (1).

15. The method as claimed in any of claims 1 to 11, in which the point and connecting markers (4) are provided on the object (1) by sticking self-adhesive films having the markers applied thereto onto the surface of the object (1).

16. An arrangement for detecting the 3D shape of an object by photogrammetry, comprising an imaging system for obtaining photogrammetric images of different views of the object (1) and a system for processing and evaluating the images and for determining the 3D shape of the object, **characterized in that** the arrangement further includes a plurality of photogrammetric point markers and a plurality of connecting markers (4) on the surface of the object, each connecting marker connecting a subset of the plurality of point markers with each other, with at least two different types of point markers existing that differ from each other in their optical configuration, and some of the point markers provided along a connecting marker (4) being formed in such a way that the sequence of their optical configurations results in a predetermined code that characterizes the respective connecting marker.

17. The arrangement as claimed in claim 16, further including an elastic envelope (2) which has the point and connecting markers (4) applied thereto and is designed such that it can be pulled over the object (1) and adapts to the shape of the object (1).

18. The arrangement as claimed in claim 16, further including one or more self-adhesive films having the point and connecting markers (4) applied thereto and designed such that when stuck on the surface of the object (1), they are in tight-fitting contact with the object (1).

19. The arrangement as claimed in claim 16, further comprising a projection system designed to be used for projecting the point and connecting markers (4) onto the object (1).

20. The method as claimed in any of claims 1 to 15 or the arrangement as claimed in any of claims 16 to 19, in which the object is a body part of a human being.

## Revendications

1. Procédé de détermination photogrammétrique de la forme spatiale d'un objet (1), dans lequel
sur la surface de l'objet (1) sont appliquées plusieurs marques ponctuelles et plusieurs marques de liaison (4) qui relient chacune une quantité partielle des plusieurs marques ponctuelles les unes aux autres, et il y a au moins deux types différents de marques ponctuelles qui se distinguent l'une de l'autre par leur aspect optique, et
quelques-unes des marques ponctuelles appliquées le long d'une marque de liaison (4) sont réalisées de telle sorte que de la succession de leurs aspects optiques résulte un code prédéterminé caractérisant la marque de liaison respective, plusieurs prises de vue photogrammétriques de l'objet sont faites sous des vues différentes (A1, A2, ... , A8),
un traitement d'image des prises de vues est réalisé, dans lequel les marques de liaison (4) qui correspondent les unes aux autres dans les prises de vues sont d'abord associées les unes aux autres à l'aide de leur code respectif, et ensuite, les marques ponctuelles reliées les unes aux autres sont associées les unes aux autres grâce à l'association des marques de liaison dans les prises de vues; et
la forme spatiale de l'objet (1) est déterminée, à partir de l'association des marques ponctuelles, à l'aide d'un procédé d'analyse photogrammétrique.

2. Procédé selon la revendication 1, dans lequel les marques de liaison (4) sont constituées par des lignes.

3. Procédé selon la revendication 2, dans lequel les lignes sont droites.

4. Procédé selon la revendication 2, dans lequel les lignes sont courbes.

5. Procédé selon l'une des revendications 2 à 4, dans lequel les marques ponctuelles sont chacune définies par deux lignes qui se rencontrent, dont l'une appartient à une marque de liaison (4) qui relie une quantité partielle des marques ponctuelles les unes aux autres.

6. Procédé selon la revendication 5, dans lequel l'aspect optique différent des marques ponctuelles est constitué par le fait que certaines marques ponctuelles s'étendent d'un côté de la ligne formant une marque de liaison vers cette ligne et se terminent sur celle-ci, et certaines autres marques ponctuelles s'étendent de l'autre côté de la ligne formant une marque de liaison vers cette ligne et se terminent sur celle-ci, le code caractérisant la marque de liaison résultant de la succession des marques ponctuelles s'étendant d'un côté et de l'autre.

7. Procédé selon la revendication 5, dans lequel l'aspect optique différent des marques ponctuelles est constitué par le fait que certaines marques ponctuelles s'étendent d'un côté sur une ligne formant une marque de liaison et se terminent sur la ligne, et que certaines marques ponctuelles croisent la ligne formant la marque de liaison, le code caractérisant la marque de liaison résultant de la succession de marques ponctuelles qui s'étendent d'un côté et de marques ponctuelles qui se croisent.

8. Procédé selon l'une des revendications 1 à 4, dans lequel les marques ponctuelles sont formées par des cercles.

9. Procédé selon la revendication 8, dans lequel l'aspect optique différent des marques ponctuelles est constitué par le fait que les points ont différents diamètres.

10. Procédé selon l'une des revendications précédentes, dans lequel les marques ponctuelles qui forment un code sont déposées avec une surface ayant un premier type d'aspect optique et les autres marques ponctuelles, qui ne forment pas un code, sont déposées avec une surface ayant un deuxième type d'aspect se distinguant du premier type.

11. Procédé selon la revendication 10, dans lequel l'aspect optique différent des surfaces est constitué par le fait que les surfaces ont des couleurs différentes.

12. Procédé selon l'une des revendications précédentes, dans lequel l'application des marques ponctuelles et des marques de liaison (4) sur l'objet (1) est réalisée de telle sorte qu'une enveloppe élastique (2) sur laquelle les marques sont appliquées, soit enfilée sur l'objet.

13. Procédé selon l'une des revendications 1 à 11, dans lequel l'application des marques ponctuelles et des marques de liaison (4) sur l'objet (1) est réalisée de telle sorte que les marques soient appliquées en couleur sur l'objet (1).

14. Procédé selon l'une des revendications 1 à 11, dans lequel l'application des marques ponctuelles et des marques de liaison (4) sur l'objet (1) est réalisée de telle sorte que les marques soient projetées sur l'objet (1).

15. Procédé selon l'une des revendications 1 à 11, dans lequel l'application des marques ponctuelles et des marques de liaison (4) sur l'objet (1) est réalisée de telle sorte que des films autocollants, sur lesquels les marques sont appliquées, soient collés sur la surface de l'objet (1).

16. Agencement de détermination photogrammétrique de la forme spatiale d'un objet, comportant un système de prise de vues permettant de réaliser des prises de vues photogrammétriques de différentes vues de l'objet (1) et un système de traitement et d'analyse des prises de vues ainsi que de détermination de la forme spatiale de l'objet, **caractérisé en ce que** l'agencement comprend, en outre, sur la surface de l'objet plusieurs marques ponctuelles photogrammétriques et plusieurs marques de liaison (4) qui relient chacune une quantité partielle des plusieurs marques ponctuelles, au moins deux types différents de marques ponctuelles qui se distinguent l'un de l'autre par leur aspect optique étant prévus, et quelques-unes des marques ponctuelles appliquées le long d'une marque de liaison (4) étant réalisées de telle sorte que de la succession de leurs aspects optiques résulte un code prédéterminé caractérisant la marque de liaison respective.

17. Agencement selon la revendication 16, qui présente en outre une enveloppe élastique (2) sur laquelle sont appliquées les marques ponctuelles et les marques de liaison (4) et qui est réalisée de telle sorte qu'elle puisse être enfilée sur l'objet (1) et s'adapte ainsi à la forme de l'objet.

18. Agencement selon la revendication 16, qui présente, en outre, un ou plusieurs films autocollants sur lesquels les marques ponctuelles et les marques de liaison (4) sont appliquées et qui sont réalisés de telle sorte qu'ils soient étroitement liés à l'objet (1) lorsqu'ils sont collés à la surface de l'objet (1).

19. Agencement selon la revendication 16, qui comprend en outre un système de projection qui est conçu de telle sorte que les marques ponctuelles et les marques de liaison (4) puissent ainsi être projetées sur l'objet.

20. Procédé selon l'une des revendications 1 à 15 ou agencement selon l'une des revendications 16 à 19, dans lequel l'objet est une partie d'un corps humain.
